# EUROPEAN PATENT SPECIFICATION

(11) **EP 2 524 728 B1**
(45) Date of publication and mention of the grant of the patent: **21.02.2024**
(21) Application number: 12168081.3
(22) Date of filing: 15.05.2012
(51) Int. Cl.: B01L 3/00

(54) **APPARATUS FOR SECURELY PROCESSING BIOLOGICAL SAMPLE**
VORRICHTUNG ZUR SICHEREN VERARBEITUNG EINER BIOLOGISCHEN PROBE
APPAREIL DE TRAITEMENT SÉCURISÉ D'UN ÉCHANTILLON BIOLOGIQUE

(30) Priority: 18.05.2011 TW 100117468
(43) Date of publication of application: 21.11.2012
(73) Proprietor: Labturbo Biotech Corporation, Taipei City 11167 (TW)
(72) Inventor: Daf, David, Taipei, Taiwan R.O.C. (TW)
(74) Representative: 2K Patentanwälte Blasberg Kewitz & Reichel

(56) References cited:
- WO-A2-2010/075116
- US-A1- 2003 035 759
- US-A1- 2006 177 354

## Description

### FIELD OF INVENTION

The present invention is related to an apparatus for securely processing biological sample.

### BACKGROUND OF THE INVENTION

Liquid semi-permeable membrane columns (or semi-permeable membrane columns) are commonly used in laboratory for washing, separating, or purifying biological molecules, such as DNA, RNA, and proteins. Semi-permeable membrane columns that are commonly used are mostly cylindrical in shape, wherein the bottom is provided with one or more pieces of semi-permeable membranes of special purposes. The column is infused with liquid, and an adequate force is then applied to the liquid in the column, forcing the liquid out of the column through the semi-permeable membranes.

Normally, the applied force can be a centrifugal force or air pressure. When centrifugal force is used, the column is usually placed in a liquid collecting tube, and then the liquid is infused in the column. The liquid collecting tube and the column are then placed into a centrifuge. The centrifuge is turned on to spin at high speed to generate a high centrifugal force. The liquid in the column is forced out of the column through the semi-permeable membranes and collected in the liquid collecting tube. The above process is only suitable for a single column. When an operation involving numerous columns is needed, the process would become overly cumbersome as the capacity of a centrifuge is limited. When air pressure is employed as an applied force, numerous semi-permeable membrane columns can be inserted into a vacuum manifold, which has a greater capacity than a centrifuge. By applying a positive or negative air pressure, the liquid is forced out of the columns through the semi-permeable membranes and is collected in the vacuum manifold. The process of using air pressure is more convenient when operating numerous samples or continuous operations.

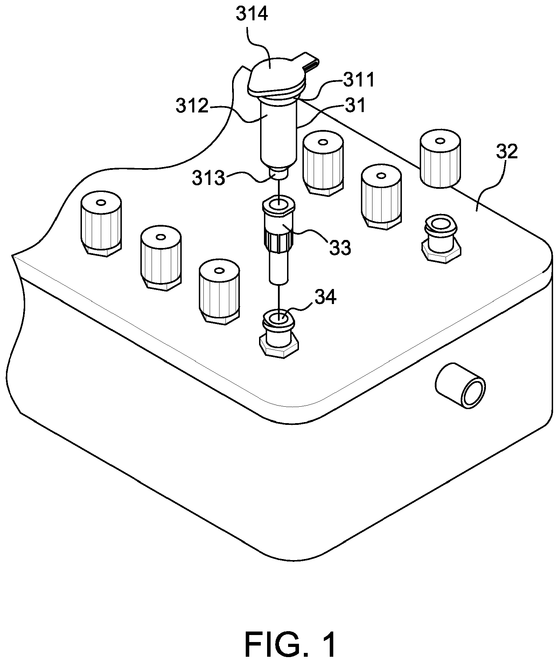

A conventional semi-permeable column 31 (see Fig. 1) generally comprises three parts: an upper cervical section 311, a middle tubular section 312, and a lower tapered section 313. The diameter of the upper cervical section 311 is larger than that of the middle tubular section 312. Some of the columns include a lid 314. The middle tubular section 312 is provided for containing liquid sample, and its internal bottom part includes one or more specific purposed semi-permeable membranes (not shown in the figure). Some of the columns have a design of lower tapered sections 313.

As shown in Figure 1, the engagement of the traditional semi-permeable membrane column 31 with a vacuum manifold 32 is in a tight insertion style: the lower tapered section 313 of the liquid semi-permeable membrane column 31 is inserted into a hole 34 of the vacuum manifold 32 directly or via an insertable adaptor column 33. The insertable adaptor column 33 is used to avoid direct insertion of the semi-permeable membrane column 31 into the hole 34 of the vacuum manifold 32, as the hole 34 of the vacuum manifold 32 may come in contact with the sample contained in the semi-permeable membrane column, and such can lead to cross contamination amongst different samples. The insertable adaptor column 33 can be of a disposable type or can be easily cleaned for repeated use. When the insertable adaptor column 33 is used, the lower tapered section 313 of the semi-permeable membrane column 31 is inserted into the insertable adaptor column 33. Then this ensemble is inserted to the hole 34 of the vacuum manifold 32, and forms the following structure from top to bottom: the semi-permeable membrane column 31 - the insertable adaptor column 33 - the vacuum manifold 32. Many applications utilize the insertable adaptor column 33, especially experiments which require no cross contamination of the samples, such as using purified nuclear acid for PCR reaction. It is therefore very important that this engagement must be tightly secured to avoid any gas leakage. Often, an operator has to hand-hold the semi-permeable membrane column 31 and the insertable adaptor column 33 to ensure tight engagement. It is likely that the operator may experience discomfort at the fingers due to this maneuver. On the other hand, as can be better understood by referring to Fig. 1, the semi-permeable membrane column 31 remains protruding outwardly from the apparatus during operation, and it is inserted into the hole 34 merely at its tip. Thus, it can easily become disengaged from the hole 34 due to any unintentional collision.

To overcome the above disadvantages, the applicant proposed an invention, which has been allowed as TW 253957. This invention provides an apparatus for processing biological sample, in which a semi-permeable column can be easily placed in the slot of a vacuum manifold. In cases where the liquid to be processed is highly contagious, an adaptor column can be used to avoid cross-contamination and to prevent the operator coming in direct contact with the liquid sample. During operation, the semi-permeable column is placed in the adaptor column, and the adaptor column is placed in the slot of the vacuum manifold. In doing so, the operator can easily operate the apparatus, and even repeated perform the operations without causing pain to the operator's fingers.

According to TW 253957, the semi-permeable column is loosely received in the slot of the vacuum manifold before air pressure or vacuum is applied. If an adaptor column is introduced between the semi-permeable column and the slot of the vacuum manifold, the gaps existing between the two columns and between the adaptor column and the slot will render that the adaptor column is loosely received in the slot and that the semi-permeable column is loosely received in the adaptor column. Due to the gaps, the semi-permeable column and/or the adaptor column can become unstable during operation, and the accuracy of tests will thus be adversely affected.

In view of this, the applicant makes improvement on TW 253957. In the improved apparatus, a seal having the effect of sealing and securing the column in position is provided such that when the semi-permeable column in placed in the slot of the vacuum manifold, air-tight condition can be maintained between the column and the slot, and that the semi-permeable column and the adaptor column will be secured in position without shaking and shifting during operation, and the accuracy of test results can be secured.

WO 2010/075116 A2 relates to nucleic acid purification and discloses a clarification/binding device for the isolation of at least one target molecule from a sample. The clarification/binding device can comprise an clarification column and a binding column. The clarification column can be configured to receive the sample. Further, the clarification column can comprise a filter configured to filter at least one non-target molecule from the sample. The binding column can be configured to receive the filtered sample from the clarification column. The binding column can comprise a binding material for binding at least one target molecule. The clarification/binding device can be configured to filter the sample and bind at least one target molecule under negative pressure. Further provided herein is an apparatus for the isolation of a target molecule from a sample. The apparatus can comprise a top plate and a vacuum manifold comprising a first chamber and a second chamber. The top plate can be configured to be used with one or both of the first vacuum chamber and the second chamber of the vacuum manifold. Further provided herein are methods of use of the clarification/binding device and the vacuum apparatus and kits comprising the clarification/binding device and vacuum apparatus.

US 2006 / 177 354 A1 discloses an apparatus for processing biological sample which are commonly used to wash, separate and purify biochemical molecules, such as DNA, RNA and protein. The apparatus comprises at least one liquid semi-permeable membrane column, at least one vacuum connected column and a vacuum manifold, which are all loosely received to each other. An air tight material such as an air tight elastic band is disposed at a position where the liquid semi-permeable membrane column, the one vacuum connected column and the vacuum manifold engage with each other, thereby when the atmospheric pressure in the liquid semi-permeable membrane column is larger than that in the vacuum manifold, the apparatus is formed and maintained in an air tight state. If a liquid sample is placed inside liquid semi-permeable membrane column, the atmospheric pressure can push the liquid out of the column through the liquid semi-permeable membranes. It is therefore much easier and convenient to assemble and disassemble the apparatus than a conventional one.

US 2003 / 035 759 A1 discloses an apparatus which is provided for simultaneous processing of multiple fluid samples. The apparatus comprises a multiple well plate for holding a plurality of sample reservoirs for simultaneous processing of multiple samples. The wells are configured in the plate in staggered rows. The well plate is dimensioned to be compatible with a vacuum manifold or positive pressure system either as a stand-alone unit or in conjunction with a separate fluid handling system. The apparatus may further comprise a sample collection plate having the same number of wells as the multiple well plate. The wells in the samples collection plate are arranged in the same staggered configuration as the wells in the well plate and are sized for holding sample collection vials.; When the well plate and sample collection plate are assembled in a manifold, the vials in the sample collection plated are aligned directly beneath the sample exits of corresponding sample reservoirs in the well plate.

### SUMMARY OF THE INVENTION

To achieve the above-mentioned objectives, the present invention provides an apparatus for processing biological sample, which comprises at least a semi-permeable column, a vacuum manifold, and at least one adaptor column. The semi-permeable column is loosely received in the adaptor column, and sealing elements made of resilient material are positioned between between the adaptor column and the slot of vacuum manifold or between the adaptor column and the semi-permeable column, such that when the adaptor column and the semi-permeable column which contains liquid sample are placed in the slot of the vacuum manifold and vacuum is applied to the vacuum manifold, the liquid sample in the semi-permeable column will be pressurized to pass through the semi-permeable membrane to achieve the desired objective.

The semi-permeable column comprises an inner portion, a top portion, and a bottom portion. The inner portion defines a first receiving space; the top portion has a first opening and a radially protruding first flange; the bottom portion has a protruding first outlet and at least one semi-permeable membrane.

The adaptor column comprises an inner portion, a top portion, and a bottom portion. The inner portion of the adaptor column defines a second receiving space. The top portion has a second opening, and a radially protruding second flange. The bottom portion has a second outlet.

The vacuum manifold comprises a base and a lid. The interior of the base defines a receiving space. The lid comprises at least one slot for receiving at least one adaptor column. The bottom of the slot has an opening in communication with the receiving space of the base.

During the operation of the apparatus of the present invention, the adaptor column is inserted into the slot of the vacuum manifold. At least one sealing element, which can be a circular gasket with a central hole, is placed at the bottom of the slot. And each of the sealing elements having an opening at its center, whereby when the adaptor column is inserted into the opening at the center of the sealing element, it can be secured in position, and when vacuum is applied to the vacuum manifold, air would not pass through between the second outlet and the sealing element, nor through between the first flange and the second sealing element The central hole allows the protruding outlet of the adaptor column to pass through while remaining secured in position to form a sealed contact when the semi-permeable column is inserted into the slot.

To facilitate persons having general knowledge in the art to better understand the technical features of the present invention, and put it into practice, the following descriptions are provided with the accompanied drawings:

### DESCRIPTIONS OF THE DRAWINGS

Fig. 1 illustrates a conventional apparatus for processing biological sample;
Fig. 2 illustrates a first non claimed example;
Fig. 3 illustrates a second non claimed example;
Fig. 4 illustrates the apparatus of the present invention; and
Fig. 5 illustrates a fourth non claimed example.

### DETAILED DESCRIPTION OF THE EMBODIMENTS

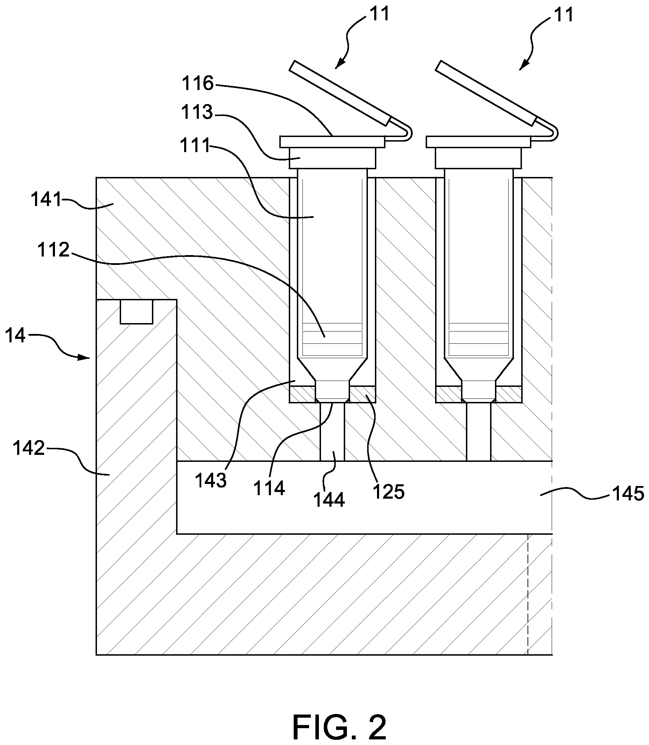

Fig. 2 illustrates a first non claimed example, wherein the semi-permeable column 11 is a conventional one. Conventional semi-permeable column is generally cylindrical in shape, which comprises an inner portion, a top portion, and a bottom portion. The inner portion defines a first receiving space 111, wherein at least one semi-permeable membrane 112 is placed. The top portion forms a first opening 116 and a first flange 113 extending radially outwardly from the top portion. A first outlet 114 is formed at the bottom portion.

As shown in Fig. 2, a sealing element 125 is positioned at the bottom of the slot 143. The center of the sealing element 125 has an opening. When the semi-permeable column is inserted into the slot 143 of the vacuum manifold 14, the sealing element 125 will allow the first outlet 114 of the semi-permeable column 11 to be inserted through its central opening. Accordingly, the semi-permeable column 11 can be secured in the slot 143 and the first outlet 114 and the sealing element 125 will form a sealed contact. When a vacuum is applied to the receiving space 145 of the vacuum manifold 14, a sealed contact between the slot 143 and the sealing element 125 will be formed and air will be prevented from flowing therebetween. The sealed contacts between the first outlet 114 and the sealing element 125 and between the slot 143 and the sealing element 125 ensure that the liquid sample in the semi-permeable column 11 is forced through only the semi-permeable membrane and out of the column under the atmospheric pressure.

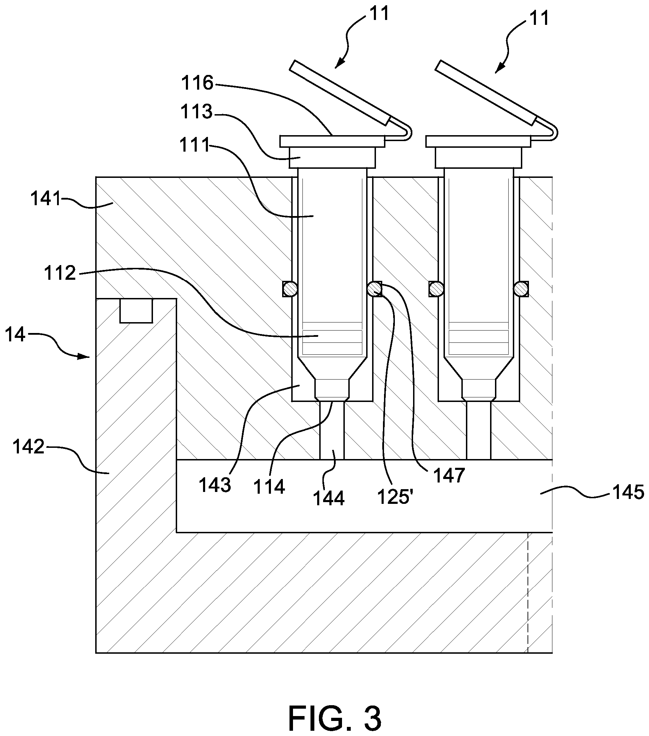

A sealing element can be also positioned on the wall of the slot 143 (shown in Fig. 3) instead of at the bottom of the slot 143 (shown in Fig. 2). The wall of the slot 143 is provided with a round groove 147 for receiving a sealing element 125', which can be an O-ring. When the semi-permeable column 11 is inserted into the slot 143, the sealing element 125' can guide to secure it in position, and when a vacuum is applied to the receiving space 145 of the vacuum manifold 14, air would not flow through between the semi-permeable column 11 and the sealing element 125'; this ensures that the sample liquid in the semi-permeable column 11 will only be forced through the semi-permeable membrane 112 and out of the column 11 under the atmospheric pressure.

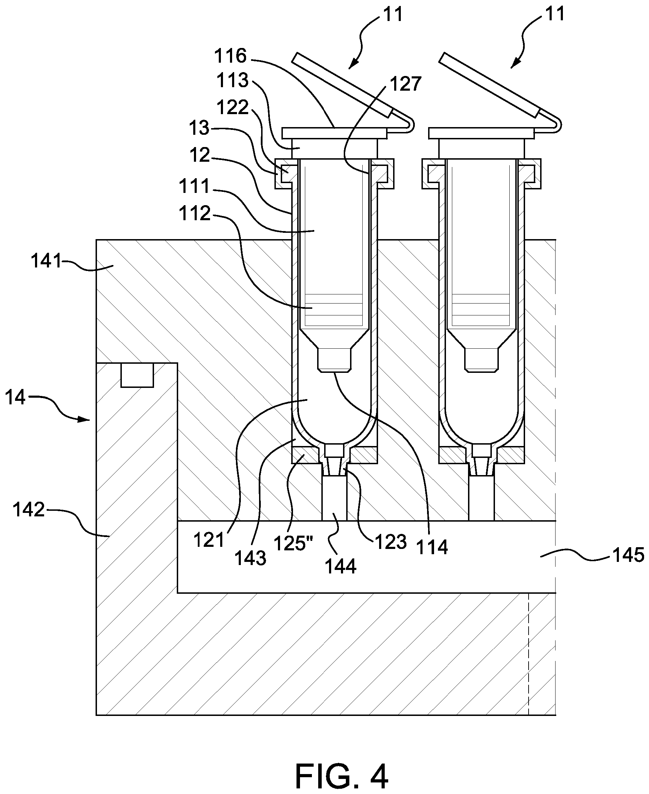

When the sample is highly contagious, an adaptor column 12 can be used and it is inserted between the semi-permeable column 11 and the slot 143, as shown in Fig. 4, to avoid cross-contamination of the sample liquid.

The adaptor column 12 generally has the shape of a column, and can be made of any appropriate materials. The adaptor column 12 comprises an inner portion, a top portion, and a bottom portion. The inner portion defines a second receiving space 121. The top portion has a second opening 127 and a second flange 122 extending radially and outwardly from the top portion. The flange 122 is fitted a resilient sealing element 13 around the circumference thereof. The bottom portion has a second outlet 123. The inner diameter of the adaptor column 12 is slightly larger than the outer diameter of the body of the semi-permeable column 11, but smaller than the diameter of the first flange 113 of the semi-permeable column 11 such that the semi-permeable column 11 can be inserted into the adaptor column 12 and be loosely received in the second receiving space 121 of the adaptor column 12 and that the first flange 113 of the top portion of the semi-permeable column 11 can rest on the sealing element 13.

Fig. 4 shows the apparatus of the present invention. With respect to the first non claimed example, the apparatus of the present invention further includes an adaptor column 12. A sealing element 13 fits around the circumference of the second flange 122 of the adaptor column 12. The sealing element 13 is preferably a resilient ring. When a semi-permeable column containing sample liquid is positioned in the adaptor column 12 and the adaptor column 12 is positioned in the slot 143 of the vacuum manifold 14, and when applying a vacuum to the vacuum manifold 14 until the pressure in the first receiving space 111 of the semi-permeable column 11 is greater than that of the receiving space 145 of the vacuum manifold 14, the first flange 113 of the semi-permeable column 11 and the sealing element 13 around the second flange 122 of the adaptor column 12 will be in a sealed contact. In this scenario, the atmospheric pressure exerting on the sample liquid in the semi-permeable column 11 will force the sample liquid to flow out of the column 11. Apart from the loosely received and air-sealed features, the adaptor column 12 has a primary function of preventing direct contact between the slot 143 of the vacuum manifold 14 and the semi-permeable column 11 in order to eliminate cross-contamination resulting from repeated use of the semi-permeable column.

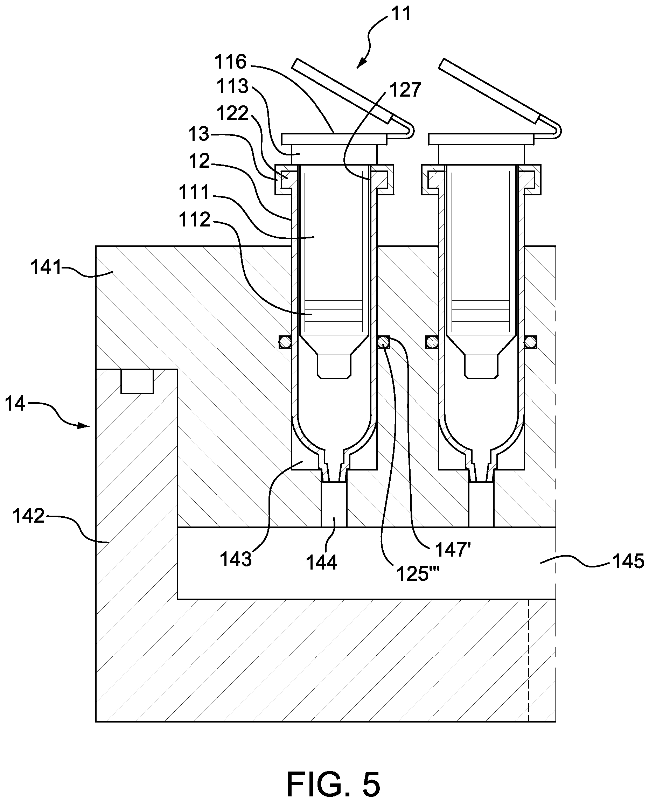

The sealing element 125" in the third embodiment shown in Fig. 4 does not necessarily need to be positioned at the bottom of the slot 143. In the fourth non claimed example shown in Fig. 5 the slot 143 similar to that of the present invention, has a circular groove 147' formed on the wall thereof. An O-ring shape sealing element 125"' is fitted to the circular groove 147'. When the adaptor column 12 is positioned into the slot 143, the sealing element 125‴ can secure the adaptor column 12 in position. When vacuum is applied to the receiving space 145 of the vacuum manifold 14, air will not pass through between the adaptor column 12 and the sealing element 125‴ nor between the first flange 113 of the semi-permeable column 11 and the sealing element 13, and thus the liquid sample in the semi-permeable column 11 will only be forced through the semi-permeable membrane 112 and out of the column under the atmospheric pressure.

In summary, the objectives of the present invention are to provide an easily assembled and disassembled apparatus for processing biological samples, to stably secure the columns in position, and to eliminate the disadvantages existing in the conventional apparatus. Since the semi-permeable column or the adaptor column has the advantage of easy insertion into and removal from the slot, the apparatus of the present invention can be used in automatic operation by robot when moving the columns is required.

The present invention provides an apparatus for securely processing biological sample is used to wash, separate, or purify biological molecules, such as DNAs, RNAs, and proteins. The apparatus comprises at least one semi-permeable membrane column, a vacuum manifold, and at least one optional adaptor column. The semi-permeable membrane column is loosely received in the slot of the vacuum manifold. The semi-permeable membrane column is loosely received in the adaptor column, and the adaptor column is loosely received in the slot of the vacuum manifold. The apparatus also comprises sealing elements which are inserted between the column and the slot. When the semi-permeable membrane column contains liquid sample and vacuum is applied to the vacuum manifold, the liquid in the semi-permeable membrane column can be drawn out of the column through the membrane. When compared with conventional apparatuses, the assembling and disassembling of the apparatus of the present invention are more convenient and the safety of operation can be enhanced.

In a first non claimed example (Fig. 2) the apparatus for securely processing biological sample, comprises:
at least one semi-permeable column, wherein the inner portion of which defines a first receiving space, the bottom of the first receiving space has at least one semi-permeable membrane, the top portion of the semi-permeable column has a first opening, and the bottom of the semi-permeable column has a first outlet;
a vacuum manifold, comprising:
   a base, the interior of which defines a receiving space; and
   a lid covering the base, comprising at least one slot, wherein the internal diameter of which is slightly larger than the outer diameter of the semi-permeable column, and the slot forms a through hole 144 at the bottom thereof for communicating with the receiving space of the base;
   at least one sealing element positioning at the bottom of the slot, and each of the sealing elements having an opening at its center, whereby when the semi-permeable column is inserted into the opening at the center of the sealing element, it can be secured in position, and when vacuum is applied to the vacuum manifold, air would not pass through between the first outlet and the sealing element.
In a second non claimed example (Fig. 3) the apparatus for securely processing biological sample, comprises:
   at least one semi-permeable column, wherein the inner portion of which defines a first receiving space, the bottom of the first receiving space has at least one semi-permeable membrane, the top portion of the semi-permeable column has a first opening, and the bottom of the semi-permeable column has a first outlet;
   a vacuum manifold, comprising:
      a base, the interior of which defines a receiving space; and
      a lid covering the base, comprising at least one slot, wherein the internal diameter of which is slightly larger than the outer diameter of the semi-permeable column, and the slot forms a through hole 144 at the bottom thereof for communicating with the receiving space of the base, and further forms at least one annular groove around the wall thereof;
      at least one sealing element in the shape of an O-ring being inserted into the at least one annular groove around the wall of the slot, wherein the inner diameter of the sealing element is slightly smaller than the outer diameter of the semi-permeable column such that when the semi-permeable column is inserted into the slot, it can be secured in position, and when vacuum is applied to the vacuum manifold, air would not pass through between the semi-permeable column and the sealing element.

In the present invention (Fig. 4) the apparatus for securely processing biological sample, comprises:
at least one semi-permeable column, wherein the inner portion of which defines a first receiving space, the bottom of the first receiving space has at least one semi-permeable membrane, the top portion of the semi-permeable column has a first opening and a flange extending radially and outwardly from the first opening, and the bottom of the semi-permeable column has a first outlet;
at least one adaptor column, wherein the interior of which defines a second receiving space, the top portion of which has a second opening and a second flange extending radially and outwardly from the second opening, the bottom portion of which has a second outlet protruding downwardly, the diameter of the second receiving space is slightly larger than the outer diameter of the semi-permeable column, the second flange having a second sealing element fitting therearound such that when the semi-permeable is inserted into the adaptor column, the first flange will rest on the second sealing element;
a vacuum manifold, comprising:
   a base, the interior of which defines a receiving space; and
   a lid covering the base, comprising at least one slot, wherein the internal diameter of which is slightly larger than the outer diameter of the adaptor column, and the slot forms a through hole 144 at the bottom thereof for communicating with the receiving space of the base;
   at least one sealing element positioning at the bottom of the slot, and each of the sealing elements having an opening at its center, whereby when the adaptor column is inserted into the opening at the center of the sealing element, it can be secured in position, and when vacuum is applied to the vacuum manifold, air would not pass through between the second outlet and the sealing element, nor through between the first flange and the second sealing element.

In a fourth non claimed example (Fig. 5) the apparatus for securely processing biological sample, comprises:
at least one semi-permeable column, wherein the inner portion of which defines a first receiving space, the bottom of the first receiving space has at least one semi-permeable membrane, the top portion of the semi-permeable column has a first opening and a flange extending radially and outwardly from the first opening, and the bottom of the semi-permeable column has a first outlet;
at least one adaptor column, wherein the interior of which defines a second receiving space, the top portion of which has a second opening and a second flange extending radially and outwardly from the second opening, the bottom portion of which has a second outlet protruding downwardly, the diameter of the second receiving space is slightly larger than the outer diameter of the semi-permeable column, the second flange having a second sealing element fitting therearound such that when the semi-permeable is inserted into the adaptor column, the first flange will rest on the second sealing element;
a vacuum manifold, comprising:
   a base, the interior of which defines a receiving space; and
   a lid covering the base, comprising at least one slot, wherein the internal diameter of which is slightly larger than the outer diameter of the adaptor column, and the slot forms a through hole 144 at the bottom thereof for communicating with the receiving space of the base, and further forms at least one annular groove formed on the wall of the slot;
   at least one sealing element in the shape of an O-ring being inserted into the at least one annular groove around the wall of the slot, wherein the inner diameter of the sealing element is slightly smaller than the outer diameter of the adaptor column such that when the adaptor column is inserted into the slot, it can be secured in position, and when vacuum is applied to the vacuum manifold, air would not pass through between the adaptor column and the sealing element and nor through between the first flange and the second sealing element.

## Claims

1. An apparatus for processing biological sample, comprising:
at least one semi-permeable column (11), wherein the inner portion of which defines a first receiving space (111), the bottom of the first receiving space has at least one semi-permeable membrane (112), the top portion of the semi-permeable column has a first opening (116) and a first flange (113) extending radially and outwardly from the first opening, and the bottom of the semi-permeable column has a first outlet (114);
at least one adaptor column (12), wherein the interior of which defines a second receiving space (121), the top portion of which has a second opening (127) and a second flange (122) extending radially and outwardly from the second opening, the bottom portion of which has a second outlet (123) protruding downwardly, the diameter of the second receiving space (121) is slightly larger than the outer diameter of the semi-permeable column (11) such that the semi-permeable column (11) can be inserted into the adaptor column (12) and be loosely received in the second receiving space (121) of the adaptor column (12), the second flange (122) having a second sealing element (13) fitting therearound such that when the semi-permeable (11) is inserted into the adaptor column (12), the first flange (113) will rest on the second sealing element (13);
a vacuum manifold (14), comprising:
a base, the interior of which defines a receiving space (145); and
a lid covering the base, comprising at least one slot (143), wherein the internal diameter of which is slightly larger than the outer diameter of the adaptor column (12) such that the adaptor column (12) is loosely received in the slot (143) of the vacuum manifold (14), and the slot (143) forms a through hole (144) at the bottom thereof for communicating with the receiving space (145) of the base;
at least one sealing element (125") is positioned at the bottom of the slot (143), and each of the sealing elements having an opening at its center, whereby when the adaptor column (12) is inserted into the opening at the center of the sealing element, it can be secured in position, and when vacuum is applied to the vacuum manifold (14), air would not pass through between the second outlet (123) and the sealing element, nor through between the first flange (113) and the second sealing element.

## Patentansprüche

1. Vorrichtung zur Verarbeitung einer biologischen Probe, umfassend:
mindestens eine halbdurchlässige Säule (11), deren innerer Abschnitt einen ersten Aufnahmeraum (111) definiert, wobei der Boden des ersten Aufnahmeraums mindestens eine halbdurchlässige Membran (112) aufweist, wobei der obere Abschnitt der halbdurchlässigen Säule eine erste Öffnung (116) und einen ersten Flansch (113) aufweist, der sich von der ersten Öffnung radial und nach außen erstreckt, und wobei der Boden der halbdurchlässigen Säule einen ersten Auslass (114) aufweist;
mindestens eine Adaptersäule (12), deren Innenraum einen zweiten Aufnahmeraum (121) definiert, dessen oberer Teil eine zweite Öffnung (127) und einen sich von der zweiten Öffnung radial nach außen erstreckenden zweiten Flansch (122) aufweist, dessen unterer Teil einen nach unten ragenden zweiten Auslass (123) aufweist, wobei der Durchmesser des zweiten Aufnahmeraums (121) geringfügig größer ist als der Außendurchmesser der halbdurchlässigen Säule (11), so dass die halbdurchlässige Säule (11) in die Adaptersäule (12) eingesetzt und in dem zweiten Aufnahmeraum (121) der Adaptersäule (12) lose aufgenommen werden kann, wobei der zweite Flansch (122) ein zweites Dichtungselement (13) aufweist, das um ihn herum passt, so dass, wenn die halbdurchlässige Säule (11) in die Adaptersäule (12) eingeführt wird, der erste Flansch (113) auf dem zweiten Dichtungselement (13) aufliegt;
einen Vakuumverteiler (14), umfassend:
einen Sockel, dessen Inneres einen Aufnahmeraum (145) definiert; und
einen die Basis abdeckenden Deckel, der mindestens einen Schlitz (143) aufweist, dessen Innendurchmesser geringfügig größer ist als der Außendurchmesser der Adaptersäule (12), so dass die Adaptersäule (12) lose in dem Schlitz (143) des Vakuumverteilers (14) aufgenommen wird, und der Schlitz (143) an seinem Boden ein Durchgangsloch (144) zur Verbindung mit dem Aufnahmeraum (145) der Basis bildet;
wobei mindestens ein Dichtungselement (125") am Boden des Schlitzes (143) angeordnet ist und jedes der Dichtungselemente in seiner Mitte eine Öffnung aufweist, wodurch die Adaptersäule (12), wenn sie in die Öffnung in der Mitte des Dichtungselements eingeführt wird, in ihrer Position gesichert werden kann und, wenn Vakuum an den Vakuumverteiler (14) angelegt wird, weder Luft zwischen dem zweiten Auslass (123) und dem Dichtungselement noch zwischen dem ersten Flansch (113) und dem zweiten Dichtungselement hindurchtreten würde.

## Revendications

1. Appareil de traitement d'un échantillon biologique, comprenant:
au moins une colonne semi-perméable (11) dont la partie intérieure définit un premier espace de réception (111), le fond du premier espace de réception comprenant au moins une membrane semi-perméable (112), la partie supérieure de la colonne semi-perméable comprenant une première ouverture (116) et une première bride (113) s'étendant radialement et vers l'extérieur depuis la première ouverture, et le fond de la colonne semi-perméable comprenant une première sortie (114);
au moins une colonne d'adaptation (12) dont l'intérieur définit un deuxième espace de réception (121), dont la partie supérieure présente une deuxième ouverture (127) et une deuxième bride (122) s'étendant radialement vers l'extérieur depuis la deuxième ouverture, dont la partie inférieure présente une deuxième sortie (123) faisant saillie vers le bas, le diamètre du deuxième espace de réception (121) étant légèrement supérieur au diamètre extérieur de la colonne semi-perméable (11), de sorte que la colonne semi-perméable (11) peut être insérée dans la colonne d'adaptation (12) et reçue de manière lâche dans le deuxième espace de réception (121) de la colonne d'adaptation (12), la deuxième bride (122) ayant un deuxième élément d'étanchéité (13) qui s'ajuste autour d'elle de sorte que, lorsque la colonne semi-perméable (11) est insérée dans la colonne d'adaptation (12), la première bride (113) repose sur le deuxième élément d'étanchéité (13);
un distributeur de vide (14), comprenant :
un socle dont l'intérieur définit un espace de réception (145); et
un couvercle recouvrant la base, qui présente au moins une fente (143) dont le diamètre intérieur est légèrement plus grand que le diamètre extérieur de la colonne d'adaptation (12), de sorte que la colonne d'adaptation (12) est reçue de manière lâche dans la fente (143) du distributeur à vide (14), et la fente (143) forme à son fond un trou traversant (144) pour la communication avec l'espace de réception (145) de la base;
dans lequel au moins un élément d'étanchéité (125") est disposé au fond de la fente (143) et chacun des éléments d'étanchéité comporte une ouverture en son centre, grâce à quoi la colonne d'adaptation (12), lorsqu'elle est insérée dans l'ouverture au centre de l'élément d'étanchéité, peut être fixée en position et, lorsque le vide est appliqué au collecteur à vide (14), l'air ne passerait pas entre la seconde sortie (123) et l'élément d'étanchéité, ni entre la première bride (113) et le second élément d'étanchéité.
